(19) Europäisches Patentamt
European Patent Office
Office européen des brevets

(11) EP 1 525 191 B1

(12) FASCICULE DE BREVET EUROPEEN

(45) Date de publication et mention de la délivrance du brevet:
**10.02.2010 Bulletin 2010/06**

(21) Numéro de dépôt: **03750839.7**

(22) Date de dépôt: **24.07.2003**

(51) Int Cl.:
***C07D 261/08*** *(2006.01)* ***C07D 275/02*** *(2006.01)*
***C07D 231/10*** *(2006.01)*

(86) Numéro de dépôt international:
**PCT/FR2003/002341**

(87) Numéro de publication internationale:
**WO 2004/011444 (05.02.2004 Gazette 2004/06)**

# (54) NOUVEAU PROCEDE DE PREPARATION DE DERIVES STYRYLE PYRAZOLES, ISOXAZOLES ET ISOTHIAZOLES

VERFAHREN ZUR HERSTELLUNG VON PYRAZOL-, ISOXAZOL- UND ISOTHIAZOL-STYRYLDERIVATEN

NOVEL METHOD FOR PREPARING STYRYL PYRAZOLE, ISOXAZOLE AND ISOTHIAZOLE DERIVATIVES

(84) Etats contractants désignés:
**AT BE BG CH CY CZ DE DK EE ES FI FR GB GR HU IE IT LI LU MC NL PT RO SE SI SK TR**

(30) Priorité: **26.07.2002 FR 0209483**

(43) Date de publication de la demande:
**27.04.2005 Bulletin 2005/17**

(73) Titulaires:
- **Centre National de la Recherche Scientifique 75794 Paris Cedex 16 (FR)**
- **Université Montpellier II 34095 Montpellier Cedex 05 (FR)**

(72) Inventeurs:
- **GLOUX, Damien F-34090 Montpellier (FR)**
- **CRITON, Marc F-34090 Montpellier (FR)**
- **MONTERO, Jean-Louis F-34270 Valflaunes (FR)**

(74) Mandataire: **Corizzi, Valérie et al Cabinet ORES, 36, rue de Saint Pétersbourg 75008 Paris (FR)**

(56) Documents cités:
**EP-A- 0 245 825**

- **DA RE P ET AL: "Structure-activity relationships in centrally stimulating xanthone derivatives. Part IV. - Dibenzo-[A,E]-cycloheptatrien-5-one and 5-ol derivatives" CHIMIE THERAPEUTIQUE, no. 1, 1973, pages 53-56, XP002236972**
- **HERZ W ET AL: "2-Vinylpyrrole and homologs" JOURNAL OF THE AMERICAN CHEMICAL SOCIETY, vol. 76, no. 2, 20 janvier 1954 (1954-01-20), pages 576-578, XP002236973**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 525 191 B1

## Description

La présente invention est relative à un nouveau procédé de synthèse de dérivés styryle isoxazoles, styryle pyrazoles et styryle isothiazoles.

La présente invention a en particulier pour objet un procédé d'obtention de composés de la famille des styryle isoxazoles, styryle pyrazoles et styryle isothiazoles en une étape de synthèse suivie d'une recristallisation en milieu basique alcoolique, faisant office de déshydratation et de purification. Ce procédé concerne en particulier l'obtention du (E)-5-[2-(3,5-Di-tert-butyl-4-hydroxyphényl)vinyl]-3-méthylisoxazole également nommé 5-[β-(4'-hydroxy-3',5'-bis(1,1-diméthyléthyl)phényl)éthényl]-3-méthylisoxazole.

Des composés styryle isoxazoles, styryle pyrazoles et styryle isothiazoles, et en particulier le (E)-5-[2-(3,5-Di-tert-butyl-4-hydroxyphényl)vinyl]-3-méthylisoxazole, ont été décrits dans le brevet EP 0 245 825. Ces composés, inhibiteurs de 5-lipoxygénase et de cyclooxygénase sont susceptibles d'être utilisés dans une composition pharmaceutique. Ils ont également des propriétés d'écran solaire. En particulier, ils sont susceptibles d'entrer dans la composition de formulations pharmaceutiques pour le traitement de l'inflammation, de l'arthrite, des ulcères, des allergies, de l'asthme, du psoriasis, d'états cardiovasculaires chez les mammifères. Ils sont également utilisés dans des compositions pour la protection contre la lumière ultraviolette.

La synthèse des dérivés de styryle isoxazole décrite dans le brevet EP 0 245 825 peut être faite selon trois approches. En particulier dans le cas du (E)-5-[2-(3,5-Di-tert-butyl-4-hydroxyphényl)vinyl]-3-méthylisoxazole, les trois schémas de synthèse suivants sont possibles :

**I** Réaction de wittig entre l'aldéhyde (3,5-di-tert-butyl-4 hydroxybenzaldéhyde) et le triphénylphosphonium du 3,5 diméthylisoxazole

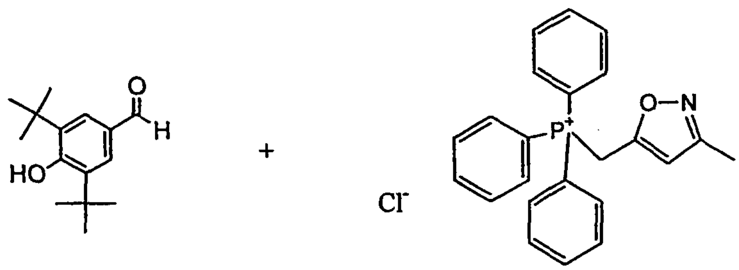

**II** Procédé en deux étapes comprenant :

- Une réaction entre l'aldéhyde (3,5-di-tert-butyl-4 hydroxybenzaldéhyde) et le sel de lithium du 3,5 diméthylisoxazole suivie d'une chromatographie sur colonne de gel de silice.
- Une seconde étape comprenant le traitement du produit résultant de la première étape avec de l'acide chlorhydrique dans du méthanol pour obtenir après purification sur colonne de gel de silice le composé souhaité, avec un rendement de 49%.

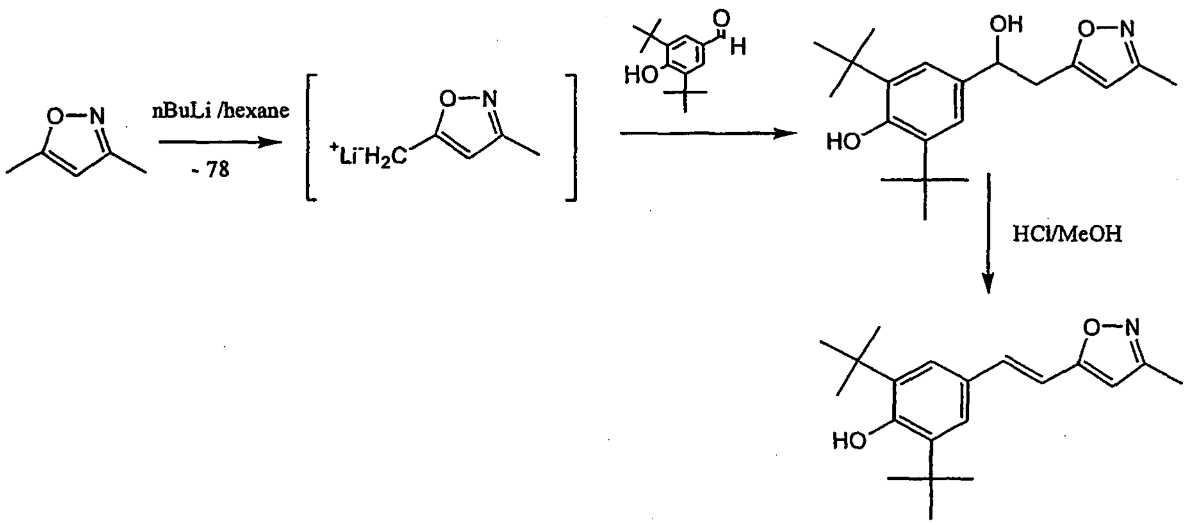


**III** Une troisième approche consiste à faire réagir l'aldéhyde (3,5-di-tert-butyl-4 hydroxybenzaldéhyde) avec un dérivé 3 carboxylate de l'isoxazole. Après condensation il est effectué une déshydratation et une décarboxylation.

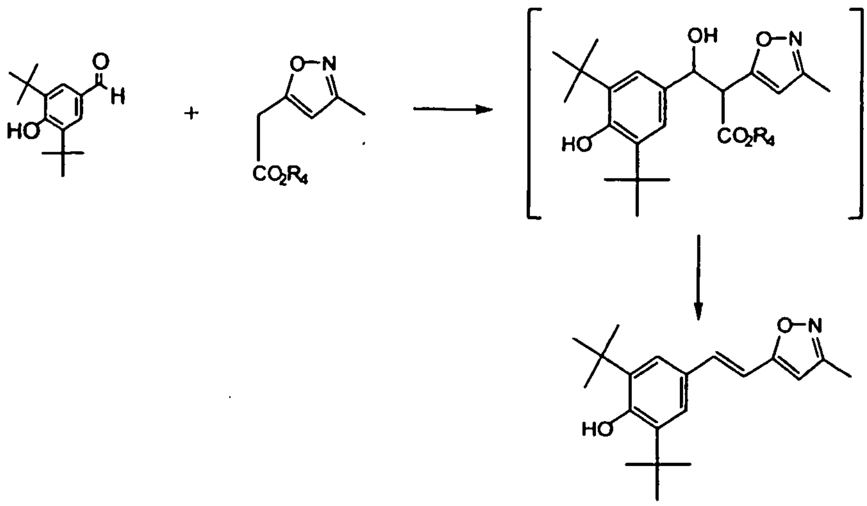

Les mêmes schémas de synthèse permettent d'accéder aux dérivés styryle pyrazoles et styryle isothiazoles.

Si les procédés de l'art antérieur permettent d'obtenir les dérivés styryle isoxazoles, styryle pyrazoles et styryle isothiazoles ils présentent l'inconvénient de nécessiter d'avoir recours à plusieurs étapes et/ou à des purifications par chromatographie afin d'obtenir un produit pur.

Le document DA RE ET AL: « Structure-activity relationships in centrally stimulating xanthone derivatives. Part IV. » CHIMIE THERAPEUTIQUE, no. 1, 1973, pages 53-56 décrit un procédé de déshydratation suivant le schéma :

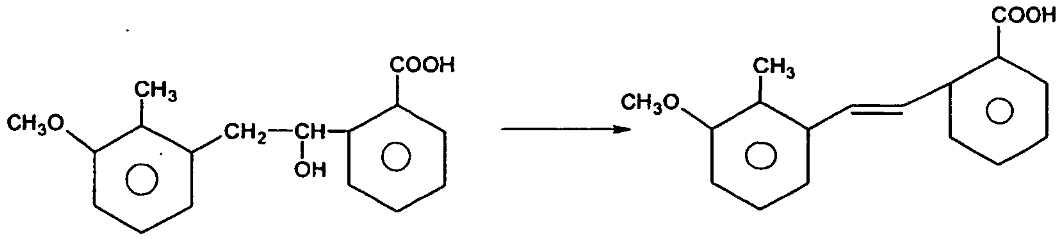

- par traitement au méthanolate de sodium dans le méthanol,
- suivi d'une purification : reprise dans l'eau, lavage à l'éther, acidification, précipitation.

Le document HERZ W ET AL: "2-Vinylpyrrole and homologues" JOURNAL OF THE AMERICAN CHEMICAL SOCIETY, vol. 76, no. 2, 20 janvier 1954 (1954-01-20), pages 576-578 décrit la réaction :

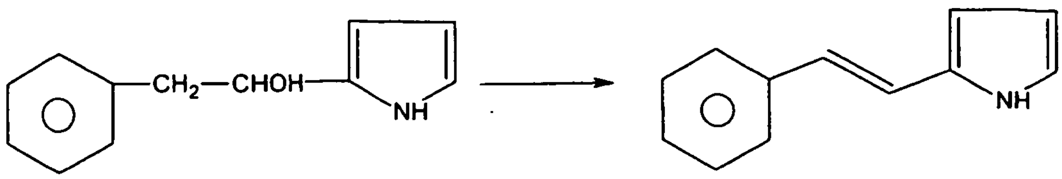

par traitement du produit de départ, sans solvant, par de la soude.

Le produit final est purifié par recristallisation et le rendement est de 22%.

La demanderesse s'est fixée pour objectif de mettre au point un procédé de préparation de dérivés styryle isoxazoles, styryle pyrazoles et styryle isothiazoles comportant un nombre limité d'étapes et permettant d'obtenir un produit présentant une pureté satisfaisante sans avoir besoin de recourir à des étapes de purification compliquées et/ou coûteuses.

Le procédé de synthèse selon l'invention s'applique à la préparation de composés répondant à la formule (I) ci-dessous

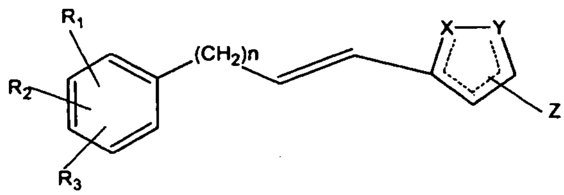

(I)

dans laquelle :

$R_1$, $R_2$, $R_3$, en position 2, 3, 4, 5, ou 6 du noyau phényl, identiques ou différents, sont choisis parmi : l'atome d'hydrogène ; les alkyles en $C_1$-$C_6$ ; les alcènyles en $C_2$-$C_6$ ; les alcynyles en $C_2$-$C_6$ ; les halogènes, les halogénoalkyles en $C_1$-$C_6$ ; -OH ; les groupements -OR', -SH, -SR', -SeH, -SeR', -C(O)R', -NHC(O)R',-C(S)R', -NHC(S)R', -CN dans lesquels R' représente un groupement choisi parmi les alkyles en $C_1$-$C_6$, les alcènyles en $C_2$-$C_6$, les alcynyles en $C_2$-$C_6$ ; les groupements-C(O)OR", -OC(O)R", -NR"R'" dans lesquels R" et R'", identiques ou différents représentent un groupement choisi parmi l'atome d'hydrogène, les alkyles en $C_1$-$C_6$, les alcènyles en $C_2$-$C_6$, les alcynyles en $C_2$-$C_6$ ;

X et Y représentent un couple d'atomes choisi parmi : ($NR_4$, N) (cycle pyrazole), (O, N) (cycle isoxazole), (S, N) (cycle isothiazole), $R_4$ étant choisi parmi : l'atome d'hydrogène ; les alkyles en $C_1$-$C_6$ ; les groupements $CH_2$-$OR_5$, les groupements $C(O)OR_5$ dans lesquels $R_5$ est choisi parmi l'atome d'hydrogène, un groupement alkyle en $C_1$-$C_6$,

un groupement benzyle ;

l'hétérocycle est relié au noyau phényl par sa position 3 ou 5 dans le cas du cycle pyrazole, par sa position 5 dans le cas des cycles isoxazole et isothiazole ;

n représente un entier choisi parmi 0, 1, 2, 3, 4, 5 et 6 ;

Z, en position 3 ou 4 du cycle isoxazole, pyrazole ou isothiazole, représente un groupement choisi parmi : l'atome d'hydrogène ; les alkyles en $C_1$-$C_6$ ; les alcényles en $C_2$-$C_6$ ; les alcynyles en $C_2$-$C_6$ ; les halogènes ; les halogénoalkyles en $C_1$-$C_6$ -OH ; les groupements -OR', -SH, -SR', -SeH, -SeR', -C(O)R', -NHC(O)R', C(S)R', -NHC(S)R', -CN dans lesquels R' représente un groupement choisi parmi les alkyles en $C_1$-$C_6$, les alcényles en $C_2$-$C_6$, les alcynyles en $C_2$-$C_6$ ; les groupements-C(O)OR", -OC(O)R", NR"R"' dans lesquels R" et R"', identiques ou différents représentent un groupement choisi parmi l'atome d'hydrogène, les alkyles en $C_1$-$C_6$, les alcényles en $C_2$-$C_6$, les alcynyles en $C_2$-$C_6$.

Les termes alkyle, alcényle et alcynyle employés dans la présente invention désignent indifféremment des radicaux linéaires ramifiés ou cycliques.

L'hétérocycle représenté par la formule ci-dessous :

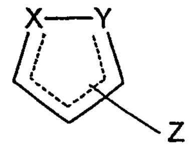

représente selon l'invention un groupement choisi parmi :

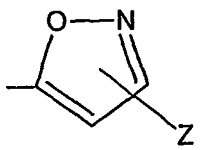

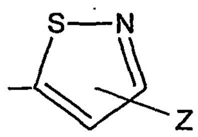

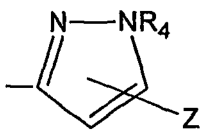

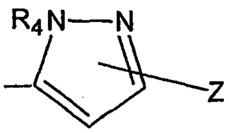

De façon préférentielle, le procédé selon la présente invention s'applique à la préparation des produits répondant à la formule (I) ci-dessus, dans lesquels une ou plusieurs des conditions ci-dessous sont remplies :

$R_1$, $R_2$, $R_3$, sont en position 3, 4, ou 5 du noyau phényl ;

$R_1$, $R_2$, $R_3$, sont choisis parmi : l'atome d'hydrogène ; les alkyles en $C_1$-$C_6$; les halogènes ; les halogénoalkyles en $C_1$-$C_6$ ; -OH ; les groupements -OR', dans lesquels R' représente un groupement choisi parmi les alkyles en $C_1$-$C_6$ ; les groupements -OC(O)R", dans lesquels R" représente un groupement choisi parmi l'atome d'hydrogène, les alkyles en $C_1$-$C_6$ ;

X=O;Y=N;

n=0 ;

Z est en position 3 de l'hétérocycle,

Z représente un groupement choisi parmi : les alkyles en $C_1$-$C_6$; les halogènes ; les halogénoalkyles en $C_1$-$C_6$.

De façon avantageuse, l'une au moins des conditions ci-dessous est remplie :

l'hétérocycle représenté par la formule :

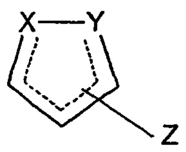

est un dérivé Z-substitué de 5-isoxazole ;
$R_1$ en position 3 est un groupement tert-butyle ;
$R_2$ en position 4 est un groupement hydroxyle ;
$R_3$ en position 5 est un groupement tert-butyle ;
Z en position 3 est un groupement méthyle.

Encore plus préférentiellement, l'invention s'applique à la préparation du (E)-5-[2-(3,5-Di-tert-butyl-4-hydroxyphényl)vinyl]-3-méthylisoxazole.

Le procédé objet de la présente invention se caractérise en ce qu'il comporte au moins une étape consistant à traiter le produit répondant à la formule (II) ci-dessous dans laquelle $R_1$, $R_2$, $R_3$ X, Y, Z et n ont la même définition que dans la formule (I) ci-dessus, dans de l'alcool en présence d'une base pour obtenir le produit de formule (I) :

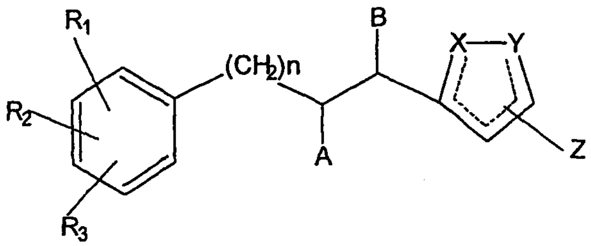

(II)

A et B étant choisis de telle sorte que l'un de A et de B est H, l'autre étant -OH.

Dans le cas des dérivés de formule (I) dans lesquels n=0, le procédé se rapproche de celui développé par Warnert Lambert dans le brevet EP 0 245 825 en ce qu'il comporte le passage par un intermédiaire commun, mais il en diffère dans le nombre d'étapes, le traitement et la purification. En effet, l'alcool (II) est soumis à un traitement en milieu basique alcoolique avec le double objectif de : 1) déshydrater l'hydroxyle (II), 2) faire cristalliser le produit (I) et par là même éliminer dans les eaux mères les impuretés, ce qui permet d'éviter une chromatographie sur colonne. Une recristallisation en milieu alcoolique peut éventuellement compléter ce procédé pour conduire au produit (I).

Par rapport aux procédés de l'art antérieur, le procédé selon l'invention se distingue par les avantages suivants : meilleur rendement, nombre d'étapes réduit, meilleure faisabilité à l'échelle industrielle.

Préférentiellement, le composé de formule (II) répond à la formule (IIa) ci-dessous dans laquelle $R_1$, $R_2$, $R_3$, X, Y et Z ont la même définition que dans la formule (1) :

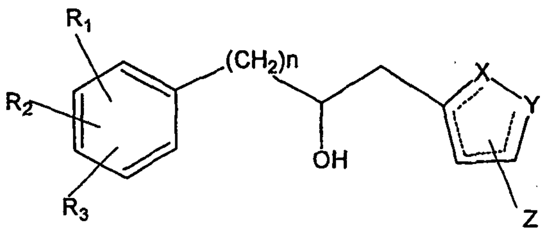

(IIa)

Cette variante représente le cas où A=OH, B=H.

Avantageusement, selon cette variante de l'invention, dans le cas ou X = O et Y = N, le composé de formule (IIa) est préparé par un procédé consistant à faire réagir l'aldéhyde (III) et le sel de lithium de l'hétérocycle (IVa) :

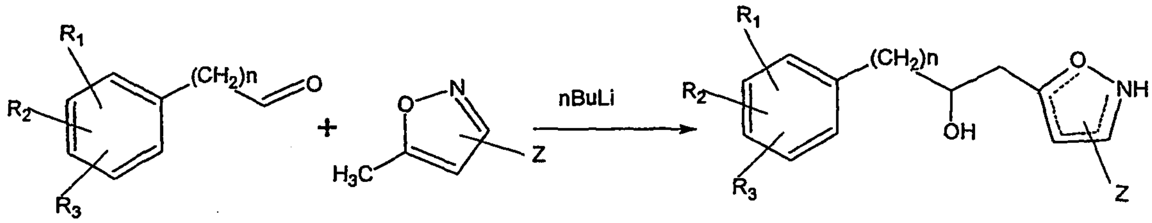


(III) (IVa) ($IIa_1$)

Plus particulièrement, dans le cas où n = 0, le composé de formule (II) est préparé par un procédé consistant à faire réagir l'aldéhyde (IIIa) et le sel de lithium du dérivé 5-méthylisoxazole (IVa) :

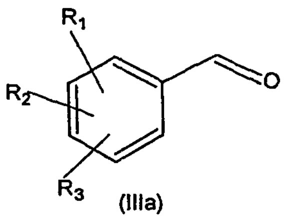

(IIIa)

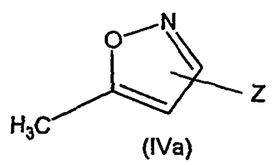

(IVa)

Dans le cas où (X,Y) représente (S, N) ou ($NR_4$, N), $R_4$ ayant la même définition que ci-dessus, on peut accéder aux molécules cibles (IIa) en faisant réagir des dérivés du phényl oxirane (V) sur le sel de lithium du dérivé 5-isothiazole (composé (IV) avec X = S et Y = N) et sur le sel de lithium du dérivé 5-pyrazole (composé (IV) avec (X,Y) = ($NR_4$, N)), suivant le schéma ci-dessous ci-dessous. De telles synthèses sont décrites en particulier dans : Ramacciotti, Alessio; Fiaschi, Rita ; Napolitano, Elio ; Tetrahedron asymetry ; 1996 ; 1101-1104 *:*

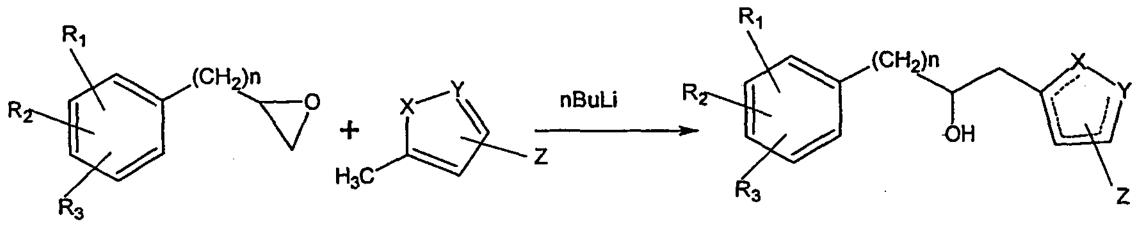


(V) (IV) (IIa)

Dans le cas où le composé de formule (II) répond à la formule (IIb) ci-dessous dans laquelle $R_1$, $R_2$, $R_3$ X, Y et Z ont la même définition que dans la formule (I) :

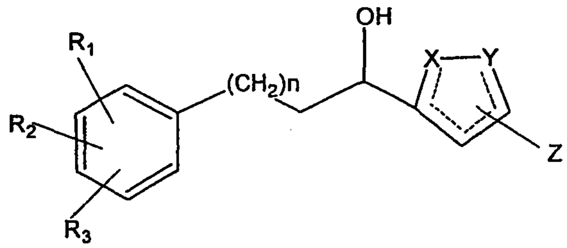

(IIb)

le produit (IIb) est avantageusement préparé par un procédé caractérisé en ce que :

dans le cas où X = O et Y = N, on fait réagir l'aldéhyde (VI) avec un sel de lithium de l'hétérocycle (VII) :

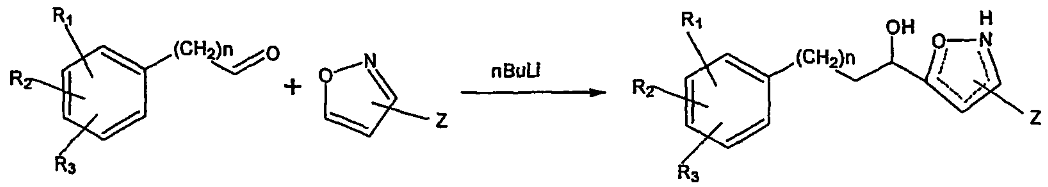


(VI) (VII) ($IIb_1$)

Dans le cas où (X,Y) représente le couple d'atomes ($NR_4$,N), $R_4$ étant tel que défini ci-dessus, on procède sensiblement suivant le schéma ci-dessus en utilisant la méthode de protection décrite par Katritky et al. : Alan R. Katritzky, Ping Lue and Kunihiko Akutagawa, Tetrahedron 45,13, (1989), 4253-4262. La fonction NH du noyau pyrazole est protégée par du formaldéhyde afin d'éviter une N-alkylation. Le pyrazole N-protégé résultant est traité par du nButyl lithium pour conduire au sel de lithium qui en présence d'aldéhyde (VI) permet d'accéder aux produits de formule (IIb)

Lorsque X = S et Y = N, le produit (IIb) est obtenu en suivant les méthodes décrites dans les articles suivants : A. J. Layton and E. Lunt J. Chem. Soc C 1968, 611-614 et Ashton, Michael J and al. J. Med. Chem., 27, 10, 1984, 1245-1253. L'isothiazole (VIII) est traité par du n-butyl lithium pour conduire au sel de lithium qui en présence d'aldéhyde (VI) permet d'accéder à une 5-alkylation pour répondre aux produits de formule ($IIb_2$).

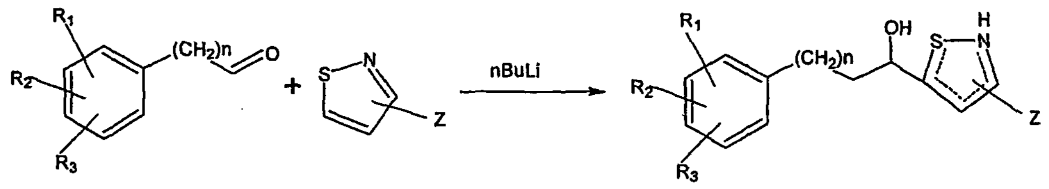


(VI) (VIII) (IIb$_2$)

Avantageusement, selon l'invention, les procédés ci-dessus s'appliquent au cas où n=0.

Avantageusement, selon l'invention le traitement du produit (II) en milieu alcoolique basique se caractérise en ce que l'alcool dans lequel se fait la déshydratation et la cristallisation est de l'éthanol, du méthanol ou de l'alcool isopropylique. Avantageusement encore, la base qui est additionnée à l'alcool est de la soude sous forme d'une solution aqueuse. De préférence la solution aqueuse de soude est une solution de concentration comprise entre 0,1M et 5M, avantageusement entre 0,5M et 4M, encore plus avantageusement entre 1M et 3M.

De façon préférentielle, le procédé comporte les étapes suivantes : dissolution du produit (II) dans de l'alcool au reflux ; addition de la base jusqu'à précipitation du composé (I) ; addition d'alcool, toujours au reflux jusqu'à solubilisation du précipité ; refroidissement de la solution qui entraîne la cristallisation de (I) ; filtration et lavage des cristaux.

**EXEMPLES**

**I Synthèse de l'(E)-5-[2-(3,5-di-tert-butyl-4-hydroxyphényl)vinyl]-3-méthylisoxazole**

1.1 Schéma Réactionnel :

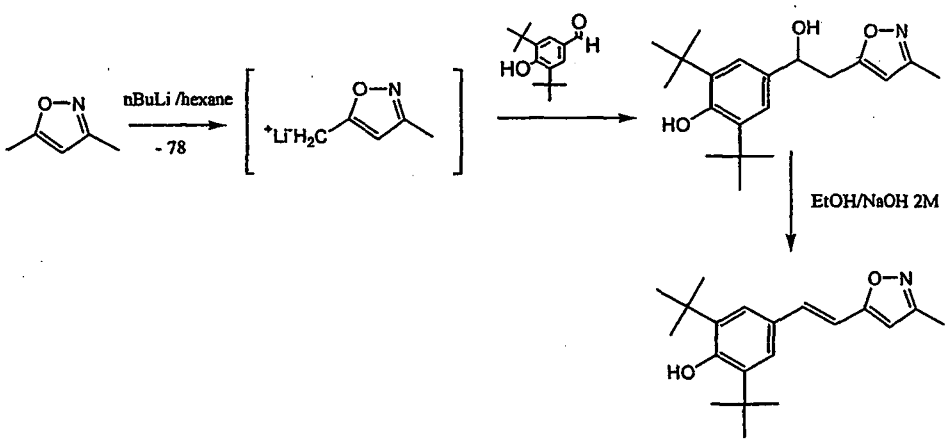


1.2 Réactifs

Les quantités de réactif sont exposées dans le tableau 1 ci-dessous

**Tableau 1**

| Réactifs | PM g/mole | Eq | mmol | Quantité |
|---|---|---|---|---|
| 3,5-diméthylisoxazole | 7.12 (d= 0.99) | 2.1 | 257.13 | 25 g |

(suite)

| Réactifs | PM g/mole | Eq | mmol | Quantité |
|---|---|---|---|---|
| n-butyllithium dans hexane | 2.5 M | 2.1 | 257.413 | 100 ml |
| 3,5-di-tert-butyl-4 hydroxybenzaldéhyde | 234.34 | | 122.577 | 28.724 g |
| THF | | | 450 ml | |

1.3 Mode opératoire :

A une solution de 3,5-diméthylisoxazole (25 g) dans le THF (200 ml) refroidie à - 78°C est ajouté (en 45 minutes) goutte à goutte le n-butyllithium (100 ml). Après 1 heures d'agitation à - 78°C; le 3,5 di-tert-butyl-4 hydroxybenzaldehyde (27.724 g) en solution dans le THF (250 ml) est ajouté goutte à goutte en 3 heures. En fin d'addition, le mélange réactionnel est laissé 2h30 sous agitation à - 78°C.

L'avancement de la réaction est suivi par chromatographie sur couche mince :

| Hexane/AcOEt | Rf aldéhyde | Rf produit (I) | Rf produit (II) |
|---|---|---|---|
| **90/10** | 0.53 | 0.4 | 0.06 |
| **80/20** | | 0.6 | 0.21 |

Lorsque l'aldéhyde a été consommé, on procède au traitement suivant :

- Concentration des solvants (THF / hexane) par évaporation sous vide,
- Reprise du milieu avec 200 ml d'acétate d'éthyle,
- Lavage par 2 fois par 100 ml $H_2O$ (pH phase aqueuse 7),
- Concentration sous vide.

La seconde partie du procédé, conformément à l'invention comporte les étapes suivantes :

a) Recristallisation/déshydratation EtOH / NaOH 2M

- Ajout de 100 ml d'éthanol, le mélange est porté au reflux
- Ajout de NaOH 2M jusqu'à précipitation du produit (I) (environ 100 ml)
- Ajout d'éthanol jusqu'à la solubilisation de (I)
- Filtration et lavage des cristaux par 100 ml d'$H_2O$ et 100 ml d'hexane.

Le (E)-5-[2-(3,5-Di-tert-butyl-4-hydroxyphenyl)vinyl]-3-methylisoxazole est obtenu avec un rendement global par rapport au produit de départ qui varie entre 50 % et 86%.

b) Eventuellement on procède ensuite à une recristallisation par EtOH (8ml/g)

Masse obtenue : 23.2 g / Rendement global : 58 %

**II - Analyse du (E)-5-[2-(3,5-di-tert-butyl-4-hydroxyphényl)vinyl]-3-méthylisoxazole**

2.1 Etude structurale

Formule développée de 1'(E)-5-[2-(3,5-di-tert-butyl-4-hydroxyphenyl)vinyl]-3-methylisoxazole :

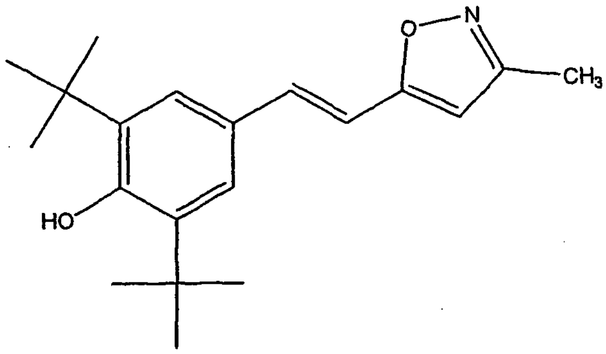

2.1.1 Spectre infra-rouge

Le spectre infra rouge est réalisé entre 4000 et 400 $cm^{-1}$ sur une pastille de KBr contenant environ 1 % d'(E)-5-[2-(3,5-di-tert-butyl-4-hydroxyphényl)vinyl]-3-méthylisoxazole
Les principaux pics observés sont :

3500 $cm^{-1}$ : $\nu_{O-H}$
2850 - 2950 $cm^{-1}$ : ν c-H (tBu, $CH_3$, CH)
1640, 1570 et 1440 $cm^{-1}$: $\nu_{C=C}$ et $\nu_{C=N}$
1230 et 1100 $cm^{-1}$: $\nu_{=C-O}$
960 $cm^{-1}$: $\nu_{N-O}$
Le spectre I.R. est conforme à la structure attendue.

2.1.2 Spectre RMN

Le spectre $^1H$ RMN est réalisé sur une solution dans le chloroforme deutéré avec un spectromètre 200 MHz.
Pics des solvants : TMS : 0 ppm; $H_2O$ : 1,55 ppm; $CHCl_3$ : 7,24 ppm.
Les pics du composé sont analysés dans le tableau 2 ci-dessous:

**Tableau 2 :**

| Déplacement chimique | Multiplicité | Intégration | Attribution |
|---|---|---|---|
| 1,45 ppm | singulet | 18 H | 2 tBu |
| 2,3 ppm | singulet | 3H | $CH_3$ en position 6 |
| 5,4 ppm | singulet | 1H | OH |
| 6,0 ppm | singulet | 1H | $H_5$ |
| 6,75 ppm | doublet | 1H | $H_4$ avec J $H_3$-$H_4$ =16 Hz |
| 7,2 ppm | doublet | 1H | $H_3$ |
| 7,3 ppm | singulet | 2 H | $H_1$ et $H_2$ |

Le spectre RMN est en conformité avec la structure attendue.

2.1.3 Spectre de masse

Le spectre de masse est réalisé en ionisation "FAB positive" avec le NBA (Alcool 3-nitrobenzylique) pour matrice.
On observe les ions suivants (tableau 3) :

**Tableau 3**

| m/z | Attribution |
|---|---|
| 313 | $M^{+\cdot}$ |
| 314 | $(M + H)^+$ |
| 627 | $(2 M + H)^+$ |

Le spectre de masse est en conformité avec la structure attendue.

2.1 Identification

2.2.1 Point de fusion

Le point de fusion a été mesuré sur différents lots, les résultats sont résumés dans le tableau 4 ci-dessous :

**Tableau 4**

| N° Lot | Aspect | Point de fusion | Pureté |
|---|---|---|---|
| MC00III17 | Grains blancs | 189°C | 99.5% |
| DG00IV53 | Cristaux blancs | 191°C | 100.7% |
| DG00IV42 | Cristaux blancs | 189°C | 100.7% |
| DG00IV52 | Flocons blancs jaunes | 190°C | 99.8% |
| MC00III11 | Grains blancs jaunes | 186°C | 97.6% |
| MC99II159 | Grains blancs jaunes | 189°C | 100.3% |
| DG01V21 | Grains jaunes beiges | 188°C | 97.4% |
| DG01V20.3 | Cristaux jaunes beige | 191°C | 96.7% |
| DG01V20.2 | Grains rosés | 187°C | 91.2% |

On constate que le point de fusion de l'(E)-5-[2-(3,5-di-tert-butyl-4-hydroxyphényl)vinyl]-3-méthylisoxazole se situe dans la plage 185-193°C.
Excepté le lot DG01V20.2, tous les lots ont une pureté supérieure à 95% quel que soit l'aspect de la poudre.

2.2.2 CCM

L'analyse CCM est réalisée dans les conditions suivantes :

- Plaque de silice $F_{254}$
- Echantillon : 100 µl de solution à 0,4 mg/ml dans MeOH
- Témoin à 1% : 20 µl de solution à 0.02 mg/ml
- Témoin à 5% : 100 µl de solution à 0.02 mg/ml
- Focalisation : MeOH
- Elution : Hexane 90/10 Acétate d'éthyle
- Migration : environ 5 cm
- Révélations : UV 254 nm et solution à 5 % de $FeCl_3$ dans HCl 0,5 M (EtOH à 95%)
- Résultats :

  - 254 nm : l'(E)-5-[2-(3,5-di-tert-butyl-4-hydroxyphényl)vinyl]-3-méthylisoxazole présente une tache de forte intensité à Rf : 0,30. Le produit de dégradation présente également une tache à Rf : 0,34.

- Révélateur $FeCl_3$ : On obtient une tache brune orangée pour l'(E)-5-[2-(3,5-di-tert-butyl-4-hydroxyphényl)vinyl]-3-méthylisoxazole au même Rf sur fond orangé pour la plaque.

2.2 Dosage

2.3.1 Perte à la dessiccation

La perte à la dessiccation permet de déterminer la teneur en eau et en solvant organique. Elle est déterminée sur 1 g si possible à l'étuve à 100-105 °C pendant 3 h ou jusqu'à poids constant.

Dans un cristallisoir préalablement desséché, on introduit une pesée d'échantillon de l'ordre de 1 g puis on dispose celui-ci dans une étuve thermostatée à 100-105°C pendant 3 h. On laisse refroidir le cristallisoir au dessiccateur jusqu'à température ambiante. On pèse le cristallisoir et on calcule la perte en % par la formule suivante :

$$\text{Perte (\%)} = \frac{(P-m) \times 100}{P}$$

Avec P : pesée introduite
m : masse après étuvage
2.3.2 Dosage par HPLC

Principe :

La technique HPLC en isocratique permet le dosage de l'(E)-5-[2-(3,5-di-tert-butyl-4-hydroxyphényl)vinyl]-3-méthylisoxazole dans des conditions de spécificité par rapport au deux précurseurs de synthèse, à une impureté de synthèse inconnue ainsi qu'au produit de dégradation par les UV.

Méthode :

La pureté de l'(E)-5-[2-(3,5-di-tert-butyl-4-hydroxyphényl)vinyl]-3-méthylisoxazole est déterminée en HPLC isocratique par étalonnage externe et exprimée par rapport à la substance sèche.

Mode opératoire :

Matériel :

Colonne : Lichrosphère C18, 5 µm, 100A°, 125x4mm + précolonne (4x4 mm)
Phase mobile : 250 H2O + 750 MeOH (solvants HPLC)
Débit : 1 ml/mn
Température ambiante
Détecteur : 240 nm
Volume d'injection : 25 µl
Intégrateur : ST=25mn, CS=2,5, SP=400, Noise=2, Sens=40, Att=4
Chaine Merck : Pompe type L-6200A
Injecteur type AS-2000A
Détecteur type L-4250
Intégrateur type D-2500

Le témoin d'(E)-5-[2-(3,5-di-tert-butyl-4-hydroxyphényl)vinyl]-3-méthylisoxazole a été synthétisé, purifié et séché au laboratoire.

Résultats :

L'analyse HPLC permet de vérifier que la pureté du produit obtenu par le procédé selon l'invention est supérieure

à 98%.

## Revendications

1. Procédé de synthèse de dérivés répondant à la formule (I) :

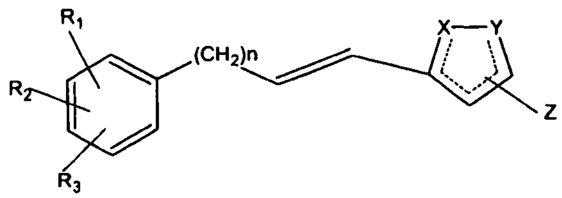

(I)

dans laquelle :

$R_1$, $R_2$, $R_3$, en position 2, 3, 4, 5, ou 6 du noyau phényl, identiques ou différents, sont choisis parmi : l'atome d'hydrogène ; les alkyles en $C_1$-$C_6$ ; les alcènyles en $C_2$-$C_6$ ; les alcynyles en $C_2$-$C_6$ ; les halogènes, les halogénoalkyles en $C_1$-$C_6$ ; -OH ; les groupements -OR', -SH, -SR', -SeH, -SeR', -C(O)R', -NHC(O)R',-C(S)R', -NHC(S)R', -CN dans lesquels R' représente un groupement choisi parmi les alkyles en $C_1$-$C_6$, les alcènyles en $C_2$-$C_6$, les alcynyles en $C_2$-$C_6$ ; les groupements-C(O)OR", -OC(O)R", -NR"R"' dans lesquels R" et R"', identiques ou différents représentent un groupement choisi parmi l'atome d'hydrogène, les alkyles en $C_1$-$C_6$, les alcènyles en $C_2$-$C_6$, les alcynyles en $C_2$-$C_6$ ;
X et Y représentent un couple d'atomes choisi parmi : ($NR_4$, N) (cycle pyrazole), (O, N) (cycle isoxazole), (S, N) (cycle isothiazole), $R_4$ étant choisi parmi : l'atome d'hydrogène ; les alkyles en $C_1$-$C_6$ ; les groupements $CH_2$-$OR_5$, les groupements $C(O)OR_5$ dans lesquels $R_5$ est choisi parmi l'atome d'hydrogène, un groupement alkyle en $C_1$-$C_6$, un groupement benzyle ; les pointillés sur la figure (I) représentent deux doubles liaisons dans l'hétérocycle,
l'hétérocycle est relié au noyau phényl par sa position 3 ou 5 dans le cas du cycle pyrazole, par sa position 5 dans le cas des cycles isoxazole et isothiazole ;
n représente un entier choisi parmi 0, 1, 2, 3, 4, 5 et 6 ;
Z, en position 3 ou 4 du cycle isoxazole, pyrazole ou isothiazole, représente un groupement choisi parmi : l'atome d'hydrogène ; les alkyles en $C_1$-$C_6$ ; les alcènyles en $C_2$-$C_6$ ; les alcynyles en $C_2$-$C_6$ ; les halogènes ; les halogénoalkyles en $C_1$-$C_6$ ; -OH ; les groupements -OR', -SH, -SR', -SeH, -SeR', -C(O)R', -NHC(O)R', C(S)R', -NHC(S)R', -CN dans lesquels R' représente un groupement choisi parmi les alkyles en $C_1$-$C_6$, les alcènyles en $C_2$-$C_6$, les alcynyles en $C_2$-$C_6$ ; les groupements-C(O)OR", -OC(O)R", -NR"R"' dans lesquels R" et R"', identiques ou différents représentent un groupement choisi parmi l'atome d'hydrogène, les alkyles en $C_1$-$C_6$, les alcènyles en $C_2$-$C_6$, les alcynyles en $C_2$-$C_6$, ce procédé étant **caractérisé en ce qu'**il comporte au moins une étape consistant à traiter le produit répondant à la formule (II) ci-dessous dans laquelle $R_1$, $R_2$, $R_3$ X, Y, Z et n ont la même définition que dans la formule (I) ci-dessus, dans de l'alcool en présence d'une base pour obtenir le produit de formule (I) sous forme cristallisée :

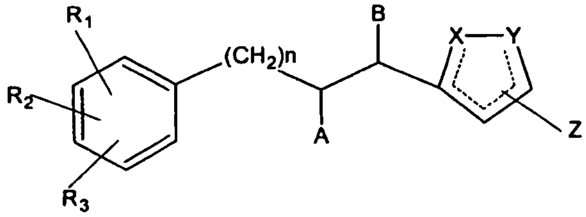

(II)

A et B étant choisis de telle sorte que l'un de A et de B est H, l'autre étant -OH.

2. Procédé selon la revendication 1, **caractérisé en ce que** l'une au moins des conditions ci-dessous est remplie :

$R_1$, $R_2$, $R_3$, sont en position 3, 4, ou 5 du noyau phényl ;
$R_1$, $R_2$, $R_3$, sont choisis parmi : l'atome d'hydrogène ; les alkyles en $C_1$-$C_6$ les halogènes ; les halogénoalkyles en $C_1$-$C_6$ -OH ; les groupements -OR', dans lesquels R' représente un groupement choisi parmi les alkyles en $C_1$-$C_6$ ; les groupements -OC(O)R", dans lesquels R" représente un groupement choisi parmi l'atome d'hydrogène, les alkyles en $C_1$-$C_6$
X=O, Y=N;
n=O ;
Z est en position 3 de l'hétérocycle,
Z représente un groupement choisi parmi : les alkyles en $C_1$-$C_6$ ; les halogènes ; les halogénoalkyles en $C_1$-$C_6$.

3. Procédé selon la revendication 1 ou la revendication 2, **caractérisé en ce que** les conditions ci-dessous sont remplies :

A=OH, B=H.

4. Procédé selon la revendication 2 ou la revendication 3, **caractérisé en ce que** l'une au moins des conditions ci-dessous est remplie :

l'hétérocycle représenté par la formule :

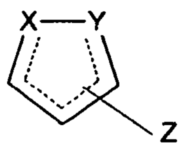

est un dérivé Z-substitué de 5-isoxazole ;
$R_1$ en position 3 est un groupement tert-butyle ;
$R_2$ en position 4 est une groupement hydroxyle ;
$R_3$ en position 5 est un groupement tert-butyle ;
Z en position 3 est un groupement méthyle.

5. Procédé selon la revendication 4, **caractérisé en ce que** le produit (I) est le (E)-5-[2-(3,5-Di-tert-butyl-4-hydroxyphenyl)vinyl]-3-methylisoxazole.

6. Procédé selon l'une quelconque des revendications précédentes, **caractérisé en ce que** l'alcool dans lequel se fait la déshydratation et la cristallisation est de l'éthanol, du méthanol ou de l'alcool isopropylique

7. Procédé selon l'une quelconque des revendications précédentes, **caractérisé en ce que** la base qui est additionnée

à l'alcool est de la soude sous forme d'une solution aqueuse.

8. Procédé selon la revendication précédente, **caractérisé en ce que** la solution aqueuse de soude est une solution de concentration comprise entre 0,1M et 5M, avantageusement entre 0,5M et 4M, encore plus avantageusement entre 1M et 3M.

9. Procédé selon l'une quelconque des revendications précédentes, **caractérisé en ce qu'**il comporte les étapes suivantes : dissolution du produit (II) dans de l'alcool au reflux ; addition de la base jusqu'à précipitation du composé (I) ; addition d'alcool, toujours au reflux jusqu'à solubilisation du précipité ; refroidissement de la solution qui entraîne la cristallisation de (I) ; filtration et lavage des cristaux.

10. Procédé selon la revendication 1 comportant une étape de préparation d'un composé de formule (II) tel que défini à la revendication 3, et dans lequel X = O et Y = N, **caractérisé en ce que** l'on fait réagir l'aldéhyde (III) et le sel de lithium de l'hétérocycle (IVa) pour obtenir le dérivé ($IIa_1$) :

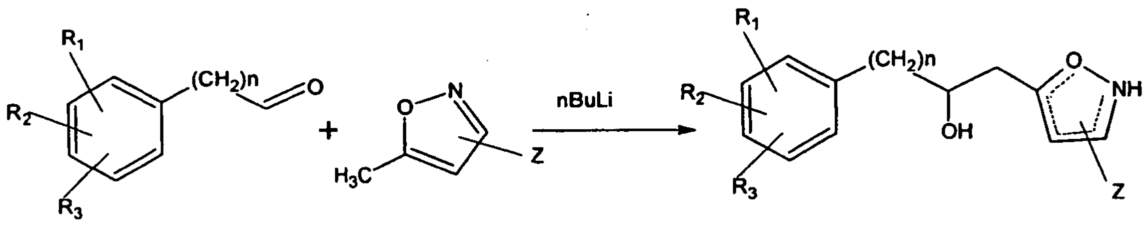


(III) (IVa) ($IIa_1$)

$R_1$, $R_2$, $R_3$ et Z ayant la même définition que dans la formule (II).

11. Procédé selon la revendication 1 comportant une étape de préparation d'un composé de formule (II) tel que défini à la revendication 3, et dans lequel (X,Y) représente (S, N) ou ($NR_4$, N), $R_4$ ayant la même définition que dans la formule (II), **caractérisé en ce que** l'on fait réagir un dérivé du phényl oxirane (V) sur le sel de lithium du dérivé 5-isothiazole (composé (IV) avec X = S et Y = N) ou sur le sel de lithium du dérivé 5-pyrazole (composé (IV) avec (X, Y) = ($NR_4$, N)), suivant le schéma ci-dessous :

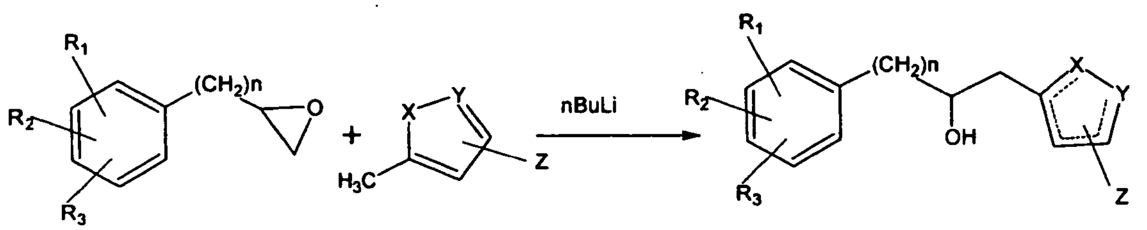


(V) (IV) (IIa)

## Claims

1. Process for the synthesis of derivatives corresponding to the formula (I):

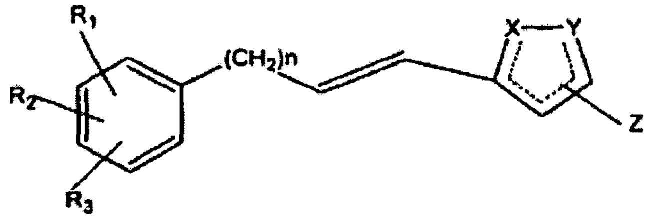

(I)

in which:

$R_1$, $R_2$, $R_3$ are in position 2, 3, 4, 5 or 6 of the phenyl nucleus, are identical or different and are chosen from: the hydrogen atom; $C_1$-$C_6$ alkyls; $C_2$-$C_6$ alkenyls; $C_2$-$C_6$-alkynyls; halogens, $C_1$-$C_6$ haloalkyls; -OH; the groups -OR', -SH, -SR', -SeH, -SeR', -C(O)R', -NHC(O)R', -C(S)R', -NHC(S)R', -CN, in which R' represents a group chosen from $C_1$-$C_6$ alkyls, $C_2$-$C_6$ alkenyls, $C_2$-$C_6$ alkynyls; the groups -C(O)OR", -OC(O)R", -NR"R"', in which R" and R"' are identical or different and represent a group chosen from the hydrogen atom, $C_1$-$C_6$ alkyls, $C_2$-$C_6$ alkenyls, $C_2$-$C_6$ alkynyls;
X and Y represent a pair of atoms chosen from: ($NR_4$, N) (pyrazole ring), (O, N) (isoxazole ring), (S, N) (isothiazole ring), $R_4$ being chosen from: the hydrogen atom, $C_1$-$C_6$ alkyls; the groups $CH_2$-$OR_5$, the groups C(O)$OR_5$, in which $R_5$ is chosen from the hydrogen atom, a $C_1$-$C_6$ alkyl group, a benzyl group; the dotted lines on figure (I) represent two double bonds in the heterocycle,
the heterocycle is bonded to the phenyl nucleus by its position 3 or 5 in the case of the pyrazole ring, by its position 5 in the case of the isoxazole and isothiazole rings;
n represents an integer chosen from 0, 1, 2, 3, 4, 5 and 6;
Z is in position 3 or 4 of the isoxazole, pyrazole or isothiazole ring and represents a group chosen from: the hydrogen atom; $C_1$-$C_6$ alkyls; $C_2$-$C_6$ alkenyls, $C_2$-$C_6$ alkynyls; halogens, $C_1$-$C_6$ haloalkyls; -OH; the groups -OR', -SH, -SR', -SeH, -SeR', -C(O)R', -NHC(O)R', -C(S)R', -NHC(S)R', -CN, in which R' represents a group chosen from $C_1$-$C_6$ alkyls, $C_2$-$C_6$ alkenyls, $C_2$-$C_6$ alkynyls; the groups -C(O)OR", -OC(O)R", -NR"R"', in which R" and R"' are identical or different and represent a group chosen from the hydrogen atom, $C_1$-$C_6$ alkyls, $C_2$-$C_6$ alkenyls, $C_2$-$C_6$ alkynyls, this process being **characterized in that** it comprises at least a stage consisting of treatment of the product corresponding to the formula (II) below, in which $R_1$, $R_2$, $R_3$, X, Y, Z and n have the same definition as in the formula (I) above, in alcohol in the presence of a base to obtain the product of the formula (I) in crystalline form:

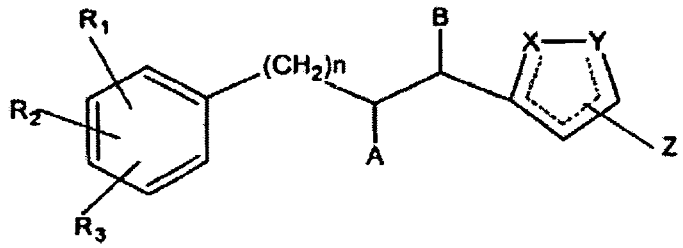

(II)

A and B being chosen such that one of A and B is H, the other being -OH.

2. Process according to claim 1, **characterized in that** at least one of the conditions below is met:

$R_1$, $R_2$, $R_3$ are in position 3, 4 or 5 of the phenyl nucleus;
$R_1$, $R_2$, $R_3$ are chosen from: the hydrogen atom; $C_1$-$C_6$ alkyls; halogens; $C_1$-$C_6$ haloalkyls; -OH; the groups

-OR', in which R' represents a group chosen from $C_1$-$C_6$ alkyls; the groups -OC(O)R", in which R" represents a group chosen from the hydrogen atom, $C_1$-$C_6$ alkyls;
X = O, Y = N;
n = 0;
Z is in position 3 of the heterocycle,
Z represents a group chosen from: $C_1$-$C_6$ alkyls; halogens; $C_1$-$C_6$ haloalkyls.

3. Process according to claim 1 or claim 2, **characterized in that** the conditions below are met:

A = OH, B = H.

4. Process according to claim 2 or claim 3, **characterized in that** at least one of the conditions below is met:

the heterocycle represented by the formula:

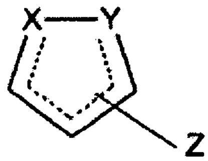

is a Z-substituted derivative of 5-isoxazole;
$R_1$ in position 3 is a tert-butyl group;
$R_2$ in position 4 is a hydroxyl group;
$R_3$ in position 5 is a tert-butyl group;
Z in position 3 is a methyl group.

5. Process according to claim 4, **characterized in that** the product (I) is (E)-5-[2-(3,5-di-tert-butyl-4-hydroxyphenyl) vinyl]-3-methylisoxazole.

6. Process according to any one of the preceding claims, **characterized in that** the alcohol in which the dehydration and the crystallization are carried out is ethanol, methanol or isopropyl alcohol.

7. Process according to any one of the preceding claims, **characterized in that** the base which is added to the alcohol is sodium hydroxide in the form of an aqueous solution.

8. Process according to the preceding claim, **characterized in that** the aqueous solution of sodium hydroxide is a solution having a concentration of between 0.1 M and 5 M, advantageously between 0.5 M and 4 M, still more advantageously between 1 M and 3 M.

9. Process according to any one of the preceding claims, **characterized in that** it comprises the following stages: dissolving of the product (II) in the alcohol under reflux; addition of the base until the compound (I) precipitates; addition of alcohol, still under reflux, until the precipitate dissolves; cooling of the solution, which causes crystallization of (I); filtration and washing of the crystals.

10. Process according to claim 1, comprising a stage of preparation of a compound of the formula (II) as defined in claim 3, and in which X = O and Y = N, **characterized in that** the aldehyde (III) and the lithium salt of the heterocycle (IVa) are reacted to obtain the derivative ($IIa_1$) :

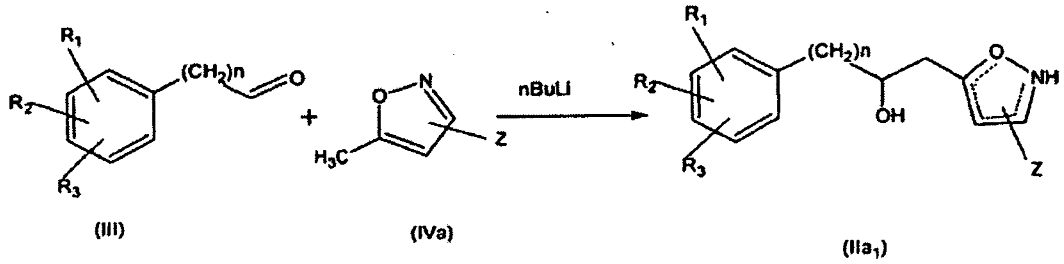


(III) (IVa) (IIa$_1$)

$R_1$, $R_2$, $R_3$ and Z having the same definition as in the formula (II).

**11.** Process according to claim 1 comprising a stage of preparation of a compound of the formula (II) as defined in claim 3 and in which (X, Y) represents (S, N) or ($NR_4$, N), $R_4$ having the same definition as in the formula (II), **characterized in that** a phenyloxirane derivative (V)is reacted on the lithium salt of the 5-isothiazole derivative (compound (IV) where X = S and Y = N) or on the lithium salt of the 5-pyrazole derivative (compound (IV) where (X, Y) = ($NR_4$, N) in accordance with the equation below:

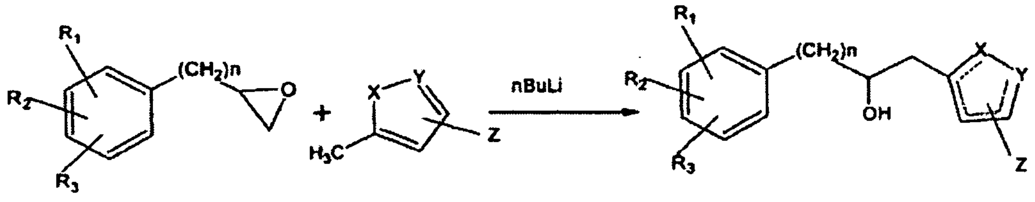


(V) (IV) (IIa)

**Patentansprüche**

**1.** Verfahren zur Synthese von Derivaten, die der Formel (I) entsprechen:

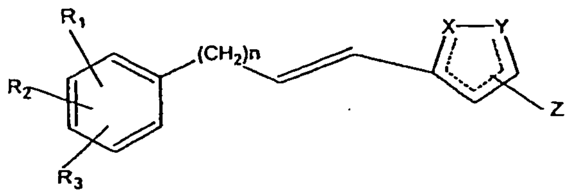

(I)

in der:

$R_1$, $R_2$, $R_3$ in Position 2, 3, 4, 5 oder 6 des Phenylkerns, die gleich oder verschieden sind, ausgewählt sind aus: einem Wasserstoffatom, ($C_1$-$C_6$)-Alkyl, ($C_2$-$C_6$)-Alkenyl, ($C_2$-$C_6$)-Alkinyl, Halogen, ($C_1$-$C_6$)-Halogenalkyl; -OH; den Gruppen -OR', -SH, -SR', -SeH, -SeR', -C(O)R', -NHC(O)R', -C(S)R', -NHC(S)R', -CN, in denen R' eine Gruppe darstellt, die ausgewählt ist aus ($C_1$-$C_6$) -Alkyl, ($C_2$-$C_6$) -Alkenyl, ($C_2$-$C_6$) -Alkinyl; den Gruppierungen -C (O)OR", -OC(O)R", -NR"R"', in denen R" und R"', die gleich oder verschieden sind, eine Gruppe darstellen, ausgewählt aus einem Wasserstoffatom, ($C_1$-$C_6$)-Alkyl, ($C_2$-$C_6$)-Alkenyl, ($C_2$-$C_6$)-Alkinyl;
X und Y ein Atompaar darstellen, ausgewählt aus: ($NR_4$, N) (Pyrazolring), (O, N) (Isoxazolring), (S, N) (Isothiazolring), wobei $R_4$ ausgewählt ist aus: einem Wasserstoffatom; ($C_1$-$C_6$) -Alkyl; $CH_2$-$OR_5$-Gruppen, C(O)

$OR_5$-Gruppen, in denen $R_5$ ausgewählt ist aus einem Wasserstoffatom, einer ($C_1$-$C_6$)-Alkylgruppe, einer Benzylgruppe; wobei die gepunkteten Linien in Figur (I) zwei Doppelbindungen in dem Heterocyclus darstellen, der Heterocyclus durch seine Position 3 oder 5 im Fall des Pyrazolrings durch seine Position 5 im Fall der Isoxazol- und Isothiazolringe an den Phenylkern gebunden ist;
n eine ganze Zahl, ausgewählt aus 0, 1, 2, 3, 4, 5 und 6, darstellt;
Z in Position 3 oder 4 des Isoxazol-, Pyrazol- oder Isothiazolrings eine Gruppe darstellt, die ausgewählt ist aus: einem Wasserstoffatom; ($C_1$-$C_6$)-Alkyl; ($C_2$-$C_6$)-Alkenyl; ($C_2$-$C_6$)-Alkinyl; Halogen; ($C_1$-$C_6$) -Halogenalkyl; -OH; den Gruppen -OR', -SH, -SR', -SeH, -SeR' , -C (O) R' , -NHC (O) R' , -C(S)R', -NHC(S)R', -CN, in denen R' eine Gruppe darstellt, ausgewählt aus ($C_1$-$C_6$) -Alkyl, ($C_2$-$C_6$) -Alkenyl, ($C_2$-$C_6$)-Alkinyl; den Gruppen -C (O) OR", -OC (O) R", -NR"R"', in denen R" und R"', die gleich oder verschieden sind, eine Gruppe darstellen, ausgewählt aus einem Wasserstoffatom, ($C_1$-$C_6$)-Alkyl, ($C_2$-$C_6$)-Alkenyl, ($C_2$-$C_6$)-Alkinyl, wobei das Verfahren **dadurch gekennzeichnet ist, dass** es wenigstens eine Stufe umfasst, die darin besteht, das Produkt, das der untenstehenden Formel (II) entspricht, in der $R_1$, $R_2$, $R_3$, X, Y, Z und n dieselbe Bedeutung wie in der obenstehenden Formel (I) haben, in Alkohol in Gegenwart einer Base behandelt wird, um das Produkt der Formel (I) in kristallisierter Form zu erhalten:

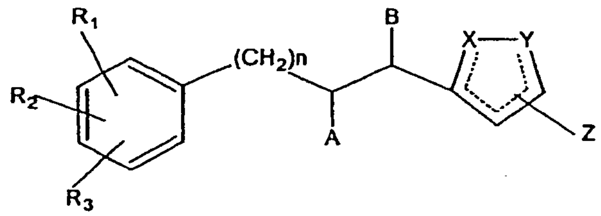

(II)

wobei A und B so ausgewählt sind, dass eines von A und B H ist, während das andere -OH ist.

2. Verfahren gemäß Anspruch 1, **dadurch gekennzeichnet, dass** wenigstens eine der unten angeführten Bedingungen erfüllt wird:

$R_1$, $R_2$, $R_3$ sind in Position 3, 4 oder 5 des Phenylkerns;
$R_1$, $R_2$, $R_3$ sind ausgewählt aus: Wasserstoffatom; ($C_1$-$C_6$)-Alkyl; Halogen; ($C_1$-$C_6$)-Halogenalkyl; -OH; -OR'-Gruppen, in denen R' eine Gruppe, ausgewählt aus ($C_1$-$C_6$) -Alkyl darstellt; -OC(O)R"-Gruppen, in denen R" eine Gruppe, ausgewählt aus Wasserstoffatom, ($C_1$-$C_6$)-Alkyl, darstellt;
X = O, Y = N;
n = 0;
Z ist in Position 3 des Heterocyclus,
Z stellt eine Gruppe dar, ausgewählt aus: ($C_1$-$C_6$)-Alkyl; Halogen; ($C_1$-$C_6$) -Halogenalkyl .

3. Verfahren gemäß Anspruch 1 oder Anspruch 2, **dadurch gekennzeichnet, dass** die untenstehenden Bedingungen erfüllt sind:

A=OH, B=H.

4. Verfahren gemäß Anspruch 2 oder Anspruch 3, **dadurch gekennzeichnet, dass** wenigstens eine der unten angeführten Bedingungen erfüllt wird:

Der durch die Formel:

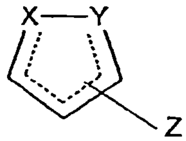

dargestellte Heterocyclus ist ein Z-substituiertes Derivat von 5-Isoxazol;
$R_1$ in Position 3 ist eine tert-Butylgruppe;
$R_2$ in Position 4 ist eine Hydroxylgruppe;
$R_3$ in Position 5 ist eine tert-Butylgruppe;
Z in Position 3 ist eine Methylgruppe.

5. Verfahren gemäß Anspruch 4, **dadurch gekennzeichnet, dass** das Produkt (I) (E)-5-[2-(3,5-Di-tert-butyl-4-hydroxyphenyl)vinyl]-3-methylisoxazol ist.

6. Verfahren nach einem der vorangehenden Ansprüche, **dadurch gekennzeichnet, dass** der Alkohol, in dem die Dehydratisierung und die Kristallisation erfolgen, Ethanol, Methanol oder Isopropylalkohol ist.

7. Verfahren gemäß einem der vorangehenden Ansprüche, **dadurch gekennzeichnet, dass** die Base, die dem Alkohol zugesetzt wird, Soda in Form einer wässrigen Lösung ist.

8. Verfahren gemäß dem vorangehenden Anspruch, **dadurch gekennzeichnet, dass** die wässrige Sodalösung eine Lösung mit einer Konzentration zwischen 0,1 M und 5 M, vorteilhafterweise zwischen 0,5 M und 4 M, noch vorteilhafter zwischen 1 M und 3 M, ist.

9. Verfahren gemäß einem der vorangehenden Ansprüche, **dadurch gekennzeichnet, dass** es die folgenden Stufen umfasst: Lösen des Produkts (II) in dem Alkohol unter Rückfluss; Zugeben der Base bis zur Präzipitation der Verbindung (I); Zugabe von Alkohol, immer unter Rückfluss bis zur Solubilisierung des Präzipitats; Abkühlen der Lösung, was die Kristallisation von (I) nach sich zieht; Filtration und Waschen der Kristalle.

10. Verfahren gemäß Anspruch 1, das eine Stufe der Herstellung einer Verbindung der Formel (II), wie sie in Anspruch 3 definiert ist und in der X = O und Y = N, umfasst, **dadurch gekennzeichnet, dass** man den Aldehyd (III) und das Lithiumsalz des Heterocyclus (IVa) miteinander reagieren lässt, wodurch das Derivat ($IIa_1$) erhalten wird:

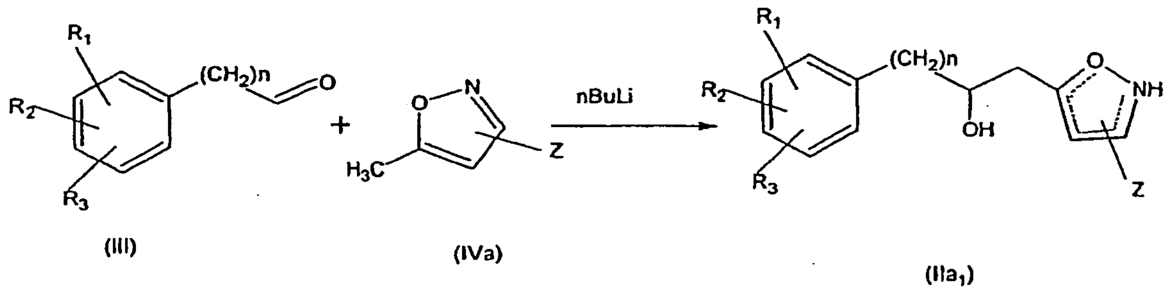


(III) (IVa) ($IIa_1$)

worin $R_1$, $R_2$, $R_3$ und Z dieselbe Definition wie in Formel (II) haben.

11. Verfahren gemäß Anspruch 1, das eine Stufe der Herstellung einer Verbindung der Formel (II), wie sie in Anspruch 3 definiert ist und in der (X, Y) für (S, N) oder ($NR_4$, N), worin $R_4$ dieselbe Bedeutung wie in Formel (II) hat, stehen, umfasst, **dadurch gekennzeichnet, dass** man ein Derivat von Phenyloxiran (V) mit dem Lithiumsalz des 5-Isothiazolderivats (Verbindung (IV) mit X = S und Y = N) oder dem Lithiumsalz des 5-Pyrazolderivats (Verbindung (IV) mit (X, Y) = ($NR_4$, N)) nach dem unten angeführten Schema reagieren lässt:

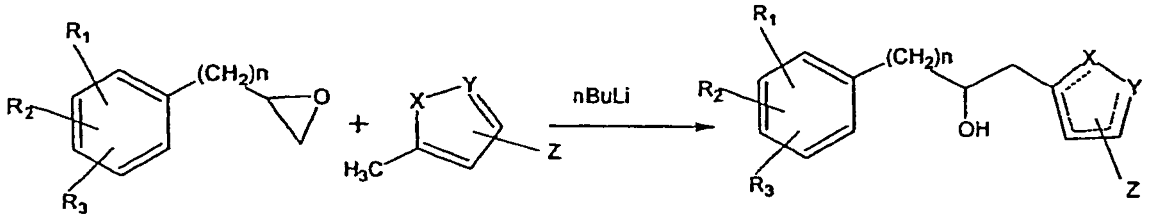


(V) (IV) (IIa)

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0245825 A


### Littérature non-brevet citée dans la description


- **DA RE et al.** Structure-activity relationships in centrally stimulating xanthone derivatives. Part IV. *CHIMIE THERAPEUTIQUE,* 1973, 53-56
- **HERZ W et al.** 2-Vinylpyrrole and homologues. *JOURNAL OF THE AMERICAN CHEMICAL SOCIETY,* 20 Janvier 1954, vol. 76 (2), 576-578
- **Ramacciotti, Alessio ; Fiaschi, Rita ; Napolitano, Elio.** *Tetrahedron asymetry,* 1996, 1101-1104
- **Alan R. Katritzky ; Ping Lue ; Kunihiko Akutagawa.** *Tetrahedron,* 1989, vol. 45 (13), 4253-4262
- **A. J. Layton ; E. Lunt.** *J. Chem. Soc C,* 1968, 611-614
- **Ashton, Michael J.** *J. Med. Chem.,* 27 Octobre 1984, 1245-1253